# EUROPEAN PATENT SPECIFICATION

(11) **EP 0 868 176 B1**
(45) Date of publication and mention of the grant of the patent: **15.08.2007**
(21) Application number: 96944681.4
(22) Date of filing: 18.12.1996
(51) Int. Cl.: A61K 31/155, A61K 45/00

(54) **AMINOGUANIDINE FOR TREATING NIDDM**
AMINOGUANIDINE ZUR BEHANDLUNG VON DIABETES TYP II
AMINOGUANIDINE POUR LE TRAITEMENT DU DIABETE DE TYPE II

(30) Priority: 22.12.1995 GB 9526330; 29.11.1996 GB 9624914
(43) Date of publication of application: 07.10.1998
(73) Proprietor: SMITHKLINE BEECHAM PLC, Brentford, Middlesex TW8 9GS (GB)
(72) Inventor: TURNER, Nicholas, Charles, Welwyn, Hertfordshire AL6 9AR (GB); PIERCY, Valerie, Welwyn, Hertfordshire AL6 9AR (GB)
(74) Representative: Rutter, Keith
(86) International application number: PCT/EP1996/005870
(87) International publication number: WO 1997/023203

(56) References cited:
- WO-A-91/12800
- WO-A-96/16301
- US-A- 5 238 963

## Description

This invention relates to a novel method for the prophylaxis of non-insulin dependant (NIDDM or Type II) diabetes, and in particular to the use of aminoguanidine or certain pharmaceutically acceptable derivatives thereof, for said prophylaxis.

Hydrazinecarboximidamide (hereinafter 'aminoguanidine') is a known compound (Journal of American Chemical Society, 57, 2730, (1935)).

Aminoguanidine is known to be an NO synthase inhibitor (Eur. J Pharmacol., 233, 119-125).

Aminoguanidine is also known to be an inhibitor of protein glycation and such activity is considered to be closely linked to the activity of aminoguanidine in the treatment of diabetic complications and other conditions associated with advanced glycosylation end products (J Carbohydrate Chem., 12(6), 731-742, (1993), Diabetes, 41, January 1992, 26-29, European Patent Application, publication number 0339496 and United States Patent numbers 5128360 and 5238963). Indeed aminoguanidine is under evaluation in animal models for the treatment of diabetic complications (Diabetes 42, 221-232 1993 and Diabetologia, 35, 946-950).

WO 91/12800 discloses the use of 3-guanidinopropionic acid in the treatment and prevention of metabolic disorders.

WO 96/16031 discloses amino guanidine carboxylates for the treatment of NIDDM and was published on 30.05.96.

To date there has been no indication that aminoguanidine or any other inhibitor of protein glycation would have a beneficial effect on Type II diabetes itself. As indicated above the emphasis has been focused upon the complications of diabetes. We have now discovered that aminoguanidine does show potential for use in the prophylaxis of Type II diabetes. In particular, aminoguanidine is indicated to delay or prevent the progression of non-insulin dependent diabetes from hyperinsulinaemia to overt diabetes. This novel and surprising effect is considered to be due to the inhibition of protein glycation by aminoguanidine.

Accordingly, the present invention provides a use of aminoguanidine according to claim 1.

When used herein a 'protein glycation inhibitor' refers to an agent which inhibits the non-enzymatic glycation or glycosylation of proteins and glycoproteins (the Maillard reaction), or which prevent the formation of irreversible advanced glycation end-products, or which prevents the crosslinking of advanced glycation end-products or which cleave advanced glycation end-product cross links.

The protein glycation inhibition activity of a compound is assessed in conventional tests such as inhibition of the glycation of haemoglobin or other suitable protein (Analytical Biochemistry;1988:175:347-360).

A suitable pharmaceutically acceptable derivative is a pharmaceutically acceptable salt, or a pharmaceutically acceptable solvate thereof.

Suitable pharmaceutically acceptable salts include acid addition salts.

Suitable acid addition salts include pharmaceutically acceptable inorganic salts such as the sulphate, nitrate, phosphate, borate, hydrochloride and hydrobromide and pharmaceutically acceptable organic acid addition salts such as acetate, tartrate, maleate, citrate, succinate, benzoate, ascorbate, methanesulphonate, a-keto glutarate and a-glycerophosphate, especially the maleate salt.

Suitable pharmaceutically acceptable solvates include hydrates.

The protein glycation inhibitors of the invention may be prepared according to conventional methods, such as the methods disclosed in the above mentioned publications, for example aminoguanidine may be prepared according to the methods disclosed in J. Amer. Chem. Soc. 57,2730, (1935).

Salts and/or solvates may be prepared and isolated according to conventional procedures.

In the above mentioned prophylaxis the aminoguanidine or a pharmaceutically acceptable derivative thereof may be administered per se or preferably as a pharmaceutical composition also comprising a pharmaceutically acceptable carrier.

As used herein the term 'pharmaceutically acceptable' embraces compounds, compositions and ingredients for both human and veterinary use: for example the term 'pharmaceutically acceptable salt' embraces a veterinarily acceptable salt.

The composition may, if desired, be in the form of a pack accompanied by written or printed instructions for use.

Usually the pharmaceutical compositions of the present invention will be adapted for oral administration, although compositions for administration by other routes, such as by injection and percutaneous absorption are also envisaged.

Particularly suitable compositions for oral administration are unit dosage forms such as tablets and capsules. Other fixed unit dosage forms, such as powders presented in sachets, may also be used.

In accordance with conventional pharmaceutical practice the carrier may comprise a diluent, filler, disintegrant, wetting agent, lubricant, colourant, flavourant or other conventional adjuvant.

Typical carriers include, for example, microcrystalline cellulose, starch, sodium starch glycollate, polyvinylpyrrolidone, polyvinylpolypyrrolidone, magnesium stearate, sodium lauryl sulphate or sucrose.

Most suitably the composition will be formulated in unit dose form. Such unit dose will normally contain an amount of the active ingredient in the range of from 0.1 to 1000 mg, more usually 0.1 to 500 mg, and more especially 0.1 to 250 mg.

Conveniently, the active ingredient may be administered as a pharmaceutical composition hereinbefore defined, and this forms a particular aspect of the present invention.

In the above mentioned treatments the active compound, may be taken in doses such as those described above, one to six times a day in a manner such that the total daily dose for a 70 kg adult will generally be in the range of from 0.1 to 6000 mg, and more usually about 1 to 1500 mg, generally about 0.5 to 10 mg. That is in the range of from 1.429 x 10⁻³ to 85.714 mg/kg/day, more usually about 1.429 x 10⁻² to 21.429 mg/kg/day, generally about 7.143 x 10⁻³ to 0.1429 mg/kg/day.

No unacceptable toxicological effects are observed when active compounds are administered in accordance with the above mentioned invention.

The following Example illustrates the invention but does not limit it in any way.

### EXAMPLE

### Methodology of dbdb mouse model

The obese db/db mouse is a genetic model of type 2 non-insulin dependent diabetes which is both insulin resistant and hyperglycaemic. Male animals were obtained at 6 weeks of age. Blood samples were taken by tail tip snip for measurement of pre-treatment blood glucose. Animals were allocated into treated and control groups such that the mean and standard deviation of the fasting blood glucose concentrations of each group was similar.

On day 0 of the study a group of obese animals and their lean litter mates were killed for measurement of baseline biochemistry and histology. In addition one group of animals (control; n = 14) were fed a standard diet and a further group received aminoguanidine (500mg/kg; n = 14) in the same diet. Animals were allowed free access to food and water and their intake measured daily. At weekly intervals 24hr urine output was also measured. Mice (n = 7) were killed at 30 days and 85 days from commencement of treatment. Blood was taken for measurement of glucose and insulin concentrations and the pancreas removed for histological analysis and for measurement of pancreatic insulin.

### Data from dbdb mouse model

Food intake and body weight gain of the control and treated groups was similar throughout the experimental period.

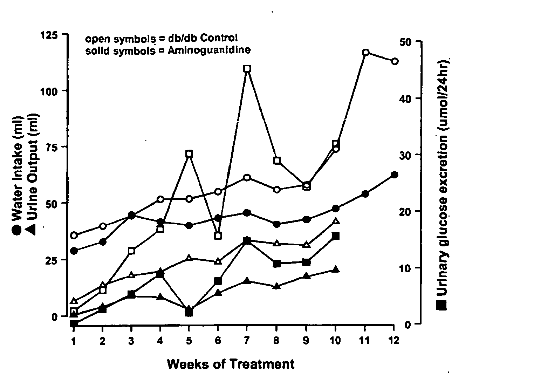

Immediately prior to dosing obese animals were normoglycaemic (blood glucose 10.4 ± 0.97mM) but were hyperinsulinaemic compared to their lean litter mates (serum isulin 127 ± 37 ng/ml in obese animals 3.05 ± 1.03 ng/ml in leans). By day 30 of the dosing period the obese control group were hyperglycaemic (blood glucose 24.9 ± 1.0 mM) and had markedly lower serum insulin levels (30.75 ± 4.3 mM) compared to the pre-treatment values. By day 85 of the treatment period, fasting blood glucose had risen to 28.1 ± 2 mM and serum insulin concentrations had fallen further, to 11.7 ± 1.8 ng/ml. Aminoguanidine attenuated the fall in fasting insulin concentrations (58.3 ±13 ng/ml on day 30, 23.3 ± 4.1 ng/ml on day 85) and
on day 85 had significantly reduced the prevailing fasting hyperglycaemia (21 ± 1.7 mM). Pancreatic insulin content of the aminoguanidine treated group of obese animals was twice that of the untreated animals (64.3 ± 17.8 ng/mg pancreas compared to 30.0 ± 2.6 ng/mg, respectively). From day 63 of the experimental period obese control animals were markedly polydypsic and polyuric compared to day 7. This increase in water intake and urine output is a characteristic of diabetes (hyperglycaemia) and was prevented by treatment with aminoguanidine (Figure 1). Similarly urinary glucose excretion increased steadily over the experimental period, in both untreated and treated animals, but from day 35 was lower in the aminoguanidine treated group (Figure 1). The development of diabetes (hyperglycaemia) was associated with changes in islet morphology, and the islets of untreated control animals were markedly hypertrophic, disorganised and had irregular boundaries. On day 85 loss of β-cells and inward collapse of the islet was evident. Islet insulin content was markedly depleted. On day 30 and 85 of the treatment period these changes in islet morphology were partially ameliorated.

## Claims

1. A use of aminoguanidine, or a pharmaceutically acceptable derivative thereof, for the manufacture of a medicament for the prophylactic treatment of Type II diabetes providing that said pharmaceutically acceptable derivative is not a compound of formula (A): 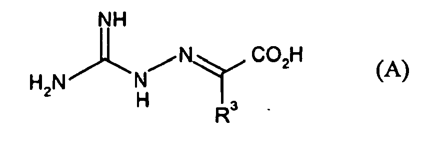 wherein R³ is hydrogen, methyl, ethyl, CH₂ phenyl, or n-hexyl.

2. A use according to claim 1, for delaying or preventing the progression from hyperinsulinaemia to hyperglycaemia.

## Patentansprüche

1. Verwendung von Aminoguanidin oder eines pharmazeutisch verträglichen Derivats davon zur Herstellung eines Medikaments zur prophylaktischen Behandlung von Typ II Diabetes, mit der Maßgabe, dass das pharmazeutisch verträgliche Derivat nicht eine Verbindung der Formel (A) ist: 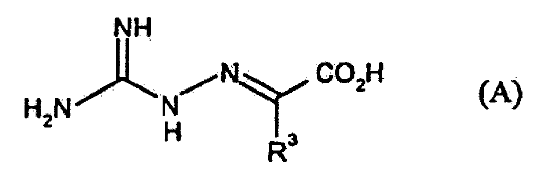 wobei R³ Wasserstoff, Methyl, Ethyl, CH₂-Phenyl oder n-Hexyl ist.

2. Verwendung nach Anspruch 1 zum Verzögern oder Verhindern des Fortschreitens von Hyperinsulinämie zu Hyperglykämie.

## Revendications

1. Utilisation d'aminoguanidine, ou d'un dérivé pharmaceutiquement acceptable de celle-ci, pour la fabrication d'un médicament destiné au traitement prophylactique du diabète de type II, à condition que ledit dérivé pharmaceutiquement acceptable ne soit pas un composé de formule (A) : 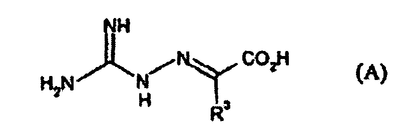 dans laquelle R³ est l'hydrogène, un groupe méthyle, éthyle, CH₂-phényle ou n-hexyle.

2. Utilisation selon la revendication 1, pour retarder ou prévenir la progression de l'hyperinsulinémie en hyperglycémie.
